Europäisches Patentamt

European Patent Office

Office européen des brevets

(19)

(11) Numéro de publication :

# 0 054 001

# B1

(12)

# FASCICULE DE BREVET EUROPÉEN

(45) Date de publication du fascicule du brevet :
03.04.85

(51) Int. Cl.⁴ : **C 12 Q   1/04, G 01 N  21/64**

(21) Numéro de dépôt : **81810474.7**

(22) Date de dépôt : **02.12.81**

(54) **Méthode pour observer le développement de micro-organismes.**

(30) Priorité : **05.12.80 CH 8993/80**

(43) Date de publication de la demande :
**16.06.82 Bulletin 82/24**

(45) Mention de la délivrance du brevet :
**03.04.85 Bulletin 85/14**

(84) Etats contractants désignés :
**AT BE CH DE FR GB IT LI LU NL SE**

(56) Documents cités :
**FR-A- 2 405 301
FR-A- 2 457 323
GB-A- 2 025 372
GB-A- 2 026 693
US-A- 3 313 290
US-A- 3 551 295
US-A- 3 772 340
US-A- 3 973 129
CHEMICAL ABSTRACTS, vol. 83, no. 17, 27 octobre
1975, page 178, colonne 2, abrégé 143442v Columbus, Ohio, US J.L. MADDOCKS et al.: "Rapid method
for identigying bacterial enzymes"**

(73) Titulaire : **BATTELLE MEMORIAL INSTITUTE
7 route de Drize
CH-1227 Carouge/Genève (CH)**

(72) Inventeur : **Ayerbe, André
Le Curly II
F-74800 Reignier (FR)**
Inventeur : **van Schouwenburg, Aaltje
14, chemin de Pierre Longue
CH-1212 Grand-Lancy (CH)**
Inventeur : **Thevoz, Michel
26, chemin des Brandards
CH-2006 Neuchatel (CH)**
Inventeur : **Moulin, Michel
24, chemin du Reposoir
CH-1007 Lausanne (CH)**
Inventeur : **Hauert, Bernard
11, route du Pont de la Fin
CH-1257 Croix de Rozon (CH)**
Inventeur : **Revillet, Georges
20, chemin François Chavaz
CH-1213 Onex (CH)**

(74) Mandataire : **Dousse, Blasco et al
7, route de Drize
CH-1227 Carouge/Genève (CH)**

EP 0 054 001 B1

Jouve, 18, rue St-Denis, 75001 Paris, France

# Description

La présente invention a pour objet l'étude du mode et de la vitesse de croissance de micro-organismes, et, notamment, une méthode pour suivre ou surveiller in vivo le développement de micro-organismes sur un milieu de culture sélectionné. Cette surveillance se fait grâce à des mesures périodiques de fluorescence dont un agent fluorescent présent dans le milieu étudié est à l'origine et dont l'intensité est en raison directe de la quantité de micro-organismes présents dans la culture soumise à l'examen au moment de la mesure. Le terme « in vivo » utilisé ici doit être bien compris comme signifiant que l'observation porte sur des micro-organismes vivants dont le développement n'est en aucune façon modifié par la mesure elle-même et uniquement conditionné par les composants du milieu de culture à l'exclusion de l'agent fluorescent lui-même. Le terme « in vivo » ne se réfère donc nullement au milieu de culture proprement dit ou à un hôte éventuel au sein duquel la culture pourrait s'effectuer.

En résumé, la présente méthode se caractérise par le fait que, lors de l'ensemencement dudit milieu de culture avec les micro-organismes en question, on ajoute à celui-ci un excès d'une substance ne présentant que peu ou pas de fluorescence propre mais capable de réagir avec lesdits micro-organismes et, par cela, de fournir ledit agent fluorescent sans porter tort audit développement, la quantité ajoutée de ladite substance constituant une réserve dudit agent fluorescent à l'état potentiel quel que soit le taux dudit développement.

En vertu de la définition qui précède, la présente méthode peut se prêter à de nombreuses applications du domaine de la microbiologie. Ainsi elle peut s'appliquer à l'analyse microbiologique et, plus particulièrement, à un procédé pour détecter et identifer des micro-organismes tels que bactéries, levures, moisissures et germes variés, de même que pour mesurer leur concentration dans des échantillons biologiques, des matières végétales, animales ou minérales, des produits alimentaires, etc.

A l'heure actuelle, la plupart des méthodes destinées à réaliser le but sus-mentionné consistent à mettre en culture un échantillon à analyser sur certains milieux nutritifs appropriés et, après une période d'incubation définie, à identifier par une technique ou une autre, le type de micro-organisme présent et à mesurer sa concentration.

Une des techniques les plus répandues pour effectuer une telle analyse consiste à ajouter au milieu de culture une ou plusieurs substances donnant une coloration en présence des micro-organismes recherchés, l'intensité de cette coloration étant, en général, proportionnelle à la concentration de ceux-ci et constituant, ainsi, un moyen de mesure de cette concentration par colorimétrie. Ainsi, à titre d'exemple, le brevet USP 3 496 066 (Berger et al.) décrit l'utilisation de composés nitro-indoliques lesquels forment des dérivés colorés en présence de bactéries contenues dans des fluides biologiques tels que le sérum et l'urine ainsi que dans des produits alimentaires, lait, eau, infusions, etc. Par mesure de la coloration, on peut estimer la concentration des micro-organismes présents dans ces matières.

Le brevet USP 4 026 767 (Shih et al.) décrit la mise en culture d'échantillons contenant des micro-organismes à analyser avec addition de sels de tétrazolium, ces derniers étant enzymatiquement réduits, en présence desdits micro-organismes, et fournissant une coloration bleue dont l'intensité, mesurée, permet d'évaluer la concentration de ces micro-organismes.

Le brevet USP 3 881 993 (Freake et al.) décrit un papier analytique poreux imprégné d'un milieu de culture, d'un fixateur (polysaccharide) et d'un réactif se colorant en rouge (formazan) au contact des micro-organismes croissant sur le milieu de culture. Cette coloration, apparaissant sur le papier, permet une estimation semi-quantitative de la quantité de micro-organismes présents.

D'autres références décrivent des méthodes similaires, à savoir le brevet autrichien n° 286 500 et les brevets allemands DP 868 080 et DOS 24 29 380, ce dernier décrivant notamment un mode de traitement chimique de bactéries conduisant à former des sites susceptibles de réagir avec certains colorants, le composé ainsi formé permettant alcrs d'identifier les bactéries en question.

Cependant, ces méthodes colorimétriques présentent certains inconvénients dus, notamment, à leur sensibilité limitée ce qui oblige à prévoir des temps de culture relativement longs (de l'ordre de 12 à 36 h) avant que des résultats significatifs puissent être obtenus. On a cherché à remédier à cet inconvénient grâce à la fluorimétrie dont la sensibilité est nettement plus élevée, notamment en raison du fait que l'intensité de la fluorescence est proportionnelle à l'intensité de la radiation d'excitation et qu'il existe, disponibles commercialement, des sources d'excitation de très forte puissance.

Ainsi, le brevet USP 3 902 971 décrit-il une méthode de détection et de mesure de micro-organismes dans l'atmosphère ou dans les eaux, cette méthode consistant à faire passer, en continu, des échantillons du milieu à analyser dans un circuit où s'effectue un mélange de l'échantillon avec un anti-sérum fluorescent de manière que les pathogènes éventuellement présents soient marqués par une réaction du type « antigène-anticorps », à amener ces pathogènes ainsi marqués sous le faisceau d'une lampe à fluorescence et à enregistrer l'intensité de la fluorescence produite à intervalles réguliers, ces mesures permettant un contrôle périodique de la contamination éventuelle de l'atmosphère ou de l'eau à analyser.

Le brevet britannique n° 1 476 006 (Block) décrit une méthode pour détecter, par fluorescence, des spécimens biologiques en faible quantité. Suivant ce procédé, on ajoute au spécimen un composé « fluorochrome », c'est-à-dire une substance qui, à l'état libre, n'est que peu ou pas fluorescente mais qui, une fois complexée avec le spécimen à déterminer, émet une fluorescence détectable. Toujours suivant ce procédé, on peut distinguer, côte à côte, deux substances fluorescentes différentes en mesurant l'intensité du signal d'émission de fluorescence à un instant donné après avoir éteint la source d'excitation, la courbe de décroissance du signal de fluorescence étant caractéristique de chacune de ces substances.

De même, il existe des procédés pour distinguer et déterminer, par mesures de fluorescence, les composants de fluides biologiques comme le sang. Ces procédés sont décrits, par exemple, dans les brevets britanniques nos 1 471 969 et 1 492 556.

Finalement, dans le brevet allemand DRP 731 205, on décrit une méthode permettant de distinguer, parmi des bactéries, levures et protozoaires, les individus encore vivants de ceux qui ont été inhibés ou tués. Cette méthode est basée sur le fait que la fluorescence des complexes entre un fluorochrome (comme par exemple le jaune d'acridine) et un micro-organisme vivant (verte) est différente de celle du complexe correspondant avec un micro-organisme mort (rouge).

Les méthodes résumées ci-dessus permettent de détecter (souvent de façon sélective), la présence de micro-organismes dans de nombreux milieux et d'évaluer, avec plus ou moins de précision, leur concentration dans ce milieu au moment de la mesure. Aucune cependant ne permet de suivre avec précision, in vivo, l'évolution de la croissance de micro-organismes donnés et d'établir, par une série de mesures à intervalles connus, le mode et le taux de croissance de tels micro-organismes dans un milieu approprié, ce mode et ce taux étant caractéristique du type de micro-organisme et de sa concentration au départ.

C'est ce but que se propose de réaliser la présente application de l'invention. En effet, celle-ci a pour objet un procédé pour détecter, identifier et doser (mesurer la concentration) dans un échantillon une ou plusieurs espèces de micro-organismes, ce procédé comprenant les étapes suivantes :

a) on ensemence avec des aliquots de l'échantillon à analyser une série de différents milieux ou compositions de culture, chacun d'entre ceux-ci étant apte à promouvoir la croissance sélective d'une souche distincte de micro-organismes, contenant au moins une substance pouvant causer, sous irradiation convenable et en présence de micro-organismes, une émission de fluorescence avec une intensité proportionnelle à la quantité de ceux-ci,

b) à intervalles de temps, on irradie ledit milieu et on mesure le degré d'intensité de cette fluorescence dans chaque milieu de culture de manière à déterminer le taux de croissance desdits micro-organismes,

c) on compare les taux ainsi mesurés avec des taux de croissance standard établis dans les mêmes milieux, pour des cultures de chacune des souches à l'état pur, de manière à identifier les micro-organismes soumis à l'analyse, et

d) on utilise les résultats des mesures pour calculer statistiquement, par exemple par extrapolation au temps zéro, la quantité des micro-organismes présents dans l'échantillon de départ.

Cette méthode, bien différente des techniques fluorométriques de l'art antérieur où le but recherché n'est que de mesurer la concentration de micro-organismes présents dans un échantillon donné à un moment donné, et non pas le mode et le taux de développement avec le temps de ce micro-organisme, est, en soi, à rapprocher des méthodes classiques suivant lesquelles on ensemence, en boîtes de Pétri, des échantillons à analyser sur des milieux de culture appropriés et, après un temps donné souvent relativement long (24 à 48 h), on observe visuellement les milieux de cultures afin de détecter l'éventuelle apparition des colonies de micro-organismes dont on soupçonnait la présence dans l'échantillon à analyser. Cependant, la méthode de l'invention présente les avantages considérables suivants par rapport aux méthodes classiques :

1) Elle est beaucoup plus sensible du fait que les signaux de fluorescence sont déjà mesurables pour des quantités de substance fluorescente extrêmement faibles.

2) Elle est plus précise, en raison de la qualité intrinsèque des mesures opto-fluorimétriques, en comparaison avec les méthodes visuelles classiques de comptage, par exemple sous microscope ou sous la loupe.

3) Elle est plus rapide car sa sensibilité permet d'effectuer les mesures relativement peu de temps après l'ensemencement des milieux de culture.

4) Elle est plus sélective car les micro-organismes peuvent être distingués non seulement par leur taux de croissance spécifique dans un milieu donné mais, dans de nombreux cas, par la nature du signal de fluorescence du complexe fluorescent, (temps de réponse, contenu chromatique et forme de la courbe d'intensité d'émission).

Parmi les substances utilisables pour engendrer un signal de fluorescence, on peut citer, de préférence, les « substrats fluorogéniques » qui comprennent des substrats (non fluorescents au départ) consommables par les micro-organismes, cette consommation ayant pour effet de libérer un agent fluorescent en quantité proportionnelle à celle des micro-organismes présents. En effet, ces substrats fluorogéniques sont constitués d'une base sucrée, aminée ou lipidique à laquelle est liée un groupement potentiellement fluorescent, cette fluorescence n'apparaissant que lors de la scission, par les enzymes produits par les micro-organismes, de ce groupement.

Parmi les substrats fluorogéniques, on peut citer les corps suivants : 4-méthyl-umbelliféryl-β-D-glucopyrannoside ; 4-méthylumbelliféryl-β-D-galactopyrannoside ; glycine-β-naphtylamide ; leucine-β-naphtylamide ; diacétate et dibutyrate de fluorescéine.

On préfère utiliser les substrats fluorogéniques car, avant libération de la portion fluorescente, ceux-ci sont totalement non fluorescents et ne provoquent pas un signal de fond dans le fluorimètre.

La présente invention peut également s'appliquer aux antibiogrammes, c'est-à-dire aux tests permettant de déterminer l'activité inhibitrice des antibiotiques (ou d'autres substances apparentées) sur la croissance des micro-organismes. Pour la mise en œuvre d'un tel test, on procède comme suit :

a) on sélectionne une souche de micro-organismes de développement positif sur un milieu de culture donné et, après avoir ajouté un substrat fluorogénique et l'antibiotique à tester à un tel milieu, on ensemence celui-ci par lesdits micro-organismes, et le maintient dans des conditions propices à la croissance de ceux-ci,

b) à intervalles de temps, on irradie ledit milieu et on mesure le degré d'intensité de la fluorescence qui en résulte ce qui permet de déterminer la vitesse de croissance desdits micro-organismes,

c) on compare le taux ainsi mesuré avec celui obtenu de manière similaire dans une culture exempte de l'antibiotique à tester de manière à déterminer le degré du pouvoir inhibiteur de celui-ci.

Bien entendu, on pourra alors répéter les étapes a) à c) ci-dessus en utilisant d'autres concentrations de l'antibiotique afin d'éventuellement déterminer, par rapport aux micro-organismes sélectionnés, quelles sont les quantités minimum d'antibiotiques nécessaires pour inhiber, de façon significative, la croissance desdits micro-organismes. Il est également évident que pour établir un spectre d'action plus complet, on répétera le test ci-dessus vis-à-vis d'autres micro-organismes. Inversement, il pourra s'agir, dans certains cas, de découvrir une substance aux propriétés antibiotiques vis-à-vis de types de micro-organismes non encore identifiés mais dont au moins un milieu favorable de culture a été déterminé. On procédera alors comme décrit ci-dessus à partir de cultures identiques desdits micro-organismes non identifiés et en faisant varier, d'un test à l'autre, la nature de l'antibiotique mis en jeu, ceci de manière à déterminer, parmi les substances inhibitrices dont on dispose, laquelle est la plus indiquée pour lutter contre une infection provoquée par lesdits micro-organismes non identifiés.

La présente invention peut encore s'appliquer au test dit de « Ames » qui consiste à déterminer si une substance quelconque est, ou non, carcinogénique. Pour la mise en œuvre d'un tel test on ensemence un milieu de culture approprié par un micro-organisme, en l'occurrence Salmonella tiphimurium (histidine moins), qui, normalement, présente un taux de croissance nul dans un tel milieu. Par contre, les mutants de S. tiphimurium (his⁻) tels que ceux provoqués par un produit cancérigène se développent positivement dans un tel milieu, ce qu'on met en évidence par la formation de colonies de tels mutants.

Dans le cas de l'application de la méthode de la présente invention au test de « Ames » on procède comme suit :

a) on ensemence de la gélose SS salmonella sans histidine contenant un substrat fluorogénique et la substance éventuellement mutagène avec une souche de S. thiphimurium (his⁻) et on maintient l'échantillon dans des conditions propices à la croissance des bactéries.

b) à intervalles de temps, on irradie ledit milieu et on mesure l'intensité de la fluorescence éventuellement émise par l'échantillon, ce qui permet de déterminer si la substance examinée est, ou non mutagène, et, le cas échéant, suivant la forme de la courbe de fluorescence, son degré d'activité en fonction de la vitesse de développement desdits mutants. Bien entendu, en utilisant le fluorimètre de l'invention, on peut soumettre simultanément au test d'Ames une série d'échantillons conforme à la description sus-mentionné et qui diffèrent les uns des autres, soit par la nature de la substance à étudier soit par la concentration de celle-ci dans le milieu de culture.

Pour mettre en œuvre la présente invention, on utilise un fluorimètre comprenant un plateau porte-réactifs circulaire tournant creusé d'alvéoles pour contenir les échantillons à analyser et les réactifs nécessaires, un dispositif optique pour émettre un rayonnement d'excitation par impulsions, ce rayonnement, de longueur d'onde choisie, étant appliqué, lors de la mesure, sur les échantillons ; un dispositif détecteur pour capter et diriger la lumière de fluorescence émise par échantillon sur un photodétecteur, et un centre électronique de contrôle, de commande, de traitement et d'affichage des résultats auquel le dispositif d'excitation et le plateau tournant sont asservis, chacune des alvéoles de celui-ci étant amenée par rotation dudit plateau en regard des dispositifs d'excitation et de détection sur commande dudit centre de contrôle, le tout de manière que, lors d'une séquence de mesures, chaque échantillon soit, tour à tour, irradié et le signal de fluorescence détecté, amplifié, traité et affiché par l'entremise dudit centre de contrôle. Le détail d'un tel fluorimètre est illustré par le dessin annexé.

La figure 1 représente schématiquement un fluorimètre à impulsions pour mesurer, à intervalles donnés, la fluorescence d'échantillons de cultures de micro-organismes.

La figure 2 est un schéma de principe de ce fluorimètre comprenant, en plus, ses organes électroniques de commande et d'enregistrement.

La figure 3 est un graphique consignant les résultats de mesures de fluorescences effectuées sur des cultures pures d'un premier micro-organisme.

La figure 4 est un graphique similaire à celui de la fig. 3 mais concernant une seconde souche de micro-organismes.

La figure 5 est un graphique consignant les résultats de mesures de fluorescence effectuées sur des cultures de staphylocoques en présence, ou non, d'antibiotiques.

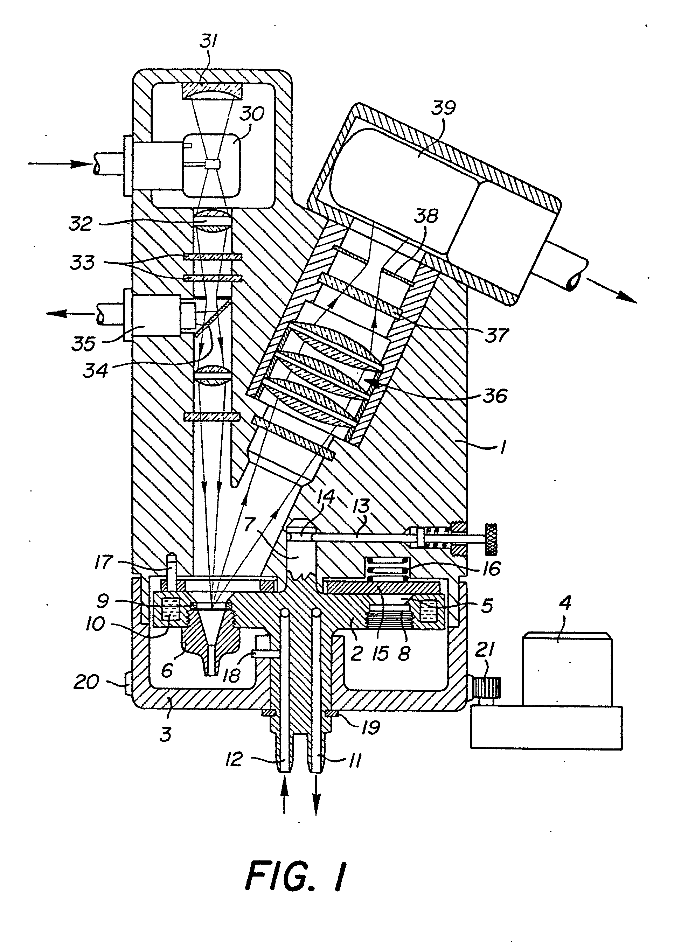

Le fluorimètre représenté à la fig. 1 comporte, dans ses grandes lignes, un boîtier 1 pour loger la partie optique de celui-ci, un plateau porte-réactifs tournant amovible 2 et, solidaire de celui-ci mais séparable à volonté, un couvercle 3 entraînable en rotation par un moteur 4.

Le plateau 2 est en un métal résistant à la corrosion et bon conducteur de la chaleur, par exemple le cuivre ou le laiton. Il est percé de trous 5 dont la partie supérieure est évasée et la partie inférieure filetée de manière qu'on puisse y adapter un embout 6 dont l'usage sera décrit plus loin. Les trous 5 sont distribués symétriquement de façon annulaire autour de l'axe 7 du plateau et leur nombre est variable, au choix, et dépend du nombre d'échantillons de milieux de culture qu'on désire analyser simultanément, comme on va le voir. En effet, dans chacun des trous 5, on peut placer, au niveau indiqué par 8, une membrane filtrante ou un papier filtre sur lequel on aura déposé la composition de culture et les micro-organismes à étudier. Cette membrane est retenue en place par un anneau clip 9 fait en un métal bon conducteur de chaleur de manière qu'une température aussi constante que possible soit maintenue au niveau des échantillons. Par ailleurs, le plateau 2 est thermostatisé par circulation d'un liquide à la température appropriée à travers le canal circulaire 10, lui-même alimenté par les conduits axiaux d'entrée et de sortie 11 et 12. L'axe 7 du plateau est maintenu dans un logement du boîtier 1 grâce à un verrou 13 solidaire de celui-ci s'engageant dans une gorge 14 dudit axe 7. Une plaque circulaire 15, prenant appui sur des ressorts 16 logés dans des compartiments du boîtier appuie à frottement doux contre le plateau 2 et fait office de pièce d'étanchéité optique, évitant que le rayonnement d'excitation ne se disperse à l'extérieur. Elle est maintenue radialement par des doigts 17. La plaque 15 assure aussi l'étanchéité du dispositif de façon à éviter l'évaporation prématurée du milieu de culture.

Le couvercle 3 est rendu solidaire du plateau 2 grâce à un ergot 18 et est maintenu en place par une clavette 19. Il est muni, sur sa périphérie, d'une denture 20 en prise avec un pignon d'entraînement 21 du moteur 4.

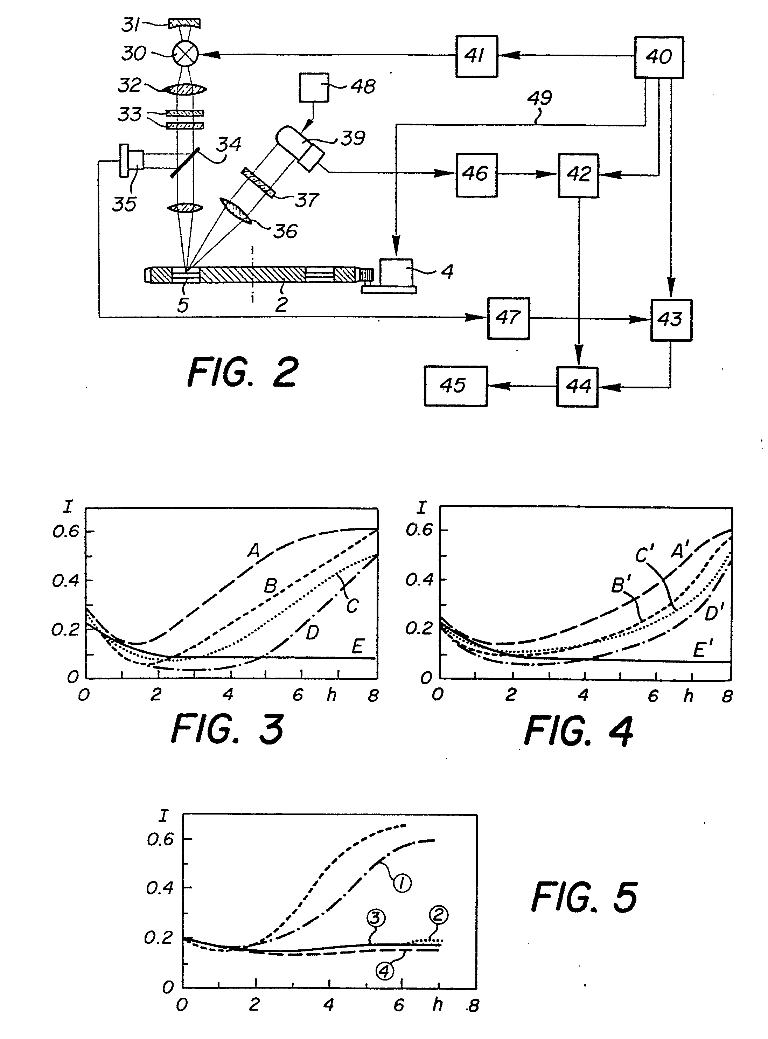

La partie optique du présent fluorimètre (voir aussi le schéma de la fig. 2) comprend essentiellement une lampe excitatrice 30 (lampe « flash ») de puissance réglable dont la lumière, réfléchie par un miroir 31, est condensée par une lentille 32 et filtrée par des filtres interchangeables 33. Ces filtres sont destinés, bien entendu, à sélectionner la ou les longueurs d'ondes choisies pour exciter la fluorescence de substances à analyser. Le rayonnement ainsi filtré traverse ensuite un miroir semi-réfléchissant 34 qui en dévie une fraction déterminée et fixe du flux total (environ 10 %) en direction d'un détecteur 35 (de référence) dont le rôle sera expliqué plus loin. Puis, après condensation et refiltrage, la lumière d'excitation parvient à l'échantillon placé dans la cuvette 5 perpendiculairement à celui-ci pour éviter toute réflexion oblique parasite. Une portion de la lumière de fluorescence (celle-ci étant radiée uniformément dans tout l'angle solide délimité par les parois du trou 5) est collimatée par le système optique de détection 36, filtrée par le filtre 37, diaphragmée par le diaphragme 38 et appliquée à un photomultiplicateur 39, celui-ci donnant naissance à un signal électrique proportionnel à l'intensité de la lumière de fluorescence émise.

Le présent fluorimètre comprend encore les organes de traitement du signal électrique et de commande suivants :

Une horloge 40 de synchronisation fournissant les impulsions de commande a, d'une part, un circuit 41 d'alimentation de la lampe flash 30 et, d'autre part, a des circuits intégrateurs 42 et 43 lesquels sont chargés de transformer les impulsions en provenance, respectivement, du photomultiplicateur 39 et du détecteur de base 35 en signaux de niveau proportionnel à la grandeur totale de ces impulsions. La présence du détecteur 35 est rendue nécessaire par le fait que les éclairs successifs émis par la lampe 30 n'ont pas tous la même énergie. En conséquence, grâce au miroir 34, le détecteur 35 recevra un pourcentage constant de l'énergie émise par chaque flash, le rapport (constant) entre l'énergie déviée et l'énergie envoyée sur l'échantillon étant, en fait, l'élément de référence fondamental du présent système. Ainsi, les signaux issus des intégrateurs 42 et 43 sont envoyés simultanément à un circuit calculateur 44 où s'effectue le calcul de rapport entre le signal de référence (proportionnel à l'impulsion d'excitation) issu, au départ, du détecteur 35 et le signal de fluorescence intégré dans l'intégrateur 42. Le résultat de ce calcul est affiché par un organe d'affichage 45 qui peut être un instrument de mesure électrique quelconque : voltmètre enregistreur, oscilloscope, affichage digital, etc. On notera que le circuit du présent fluorimètre compte encore des amplificateurs 46, 47 pour amplifier, avant intégration, les signaux de référence et de mesure, une alimentation 48 du photomultiplicateur et une ligne de commande 49 entre l'horloge 40 et le moteur 4 dont le rôle va être expliqué plus bas.

Dans la pratique habituelle de mise en œuvre de la première des applications précitées, on utilise le présent fluorimètre enregistreur comme suit : le plateau 2 et son couvercle 3 étant détachés de l'appareil, on commence par sélectionner un échantillon de souche de micro-organismes à étudier (produit à analyser ou culture de contrôle) et on dépose celui-ci sur un papier filtre ou une membrane filtrante du diamètre convenable s'insérant dans la cuvette d'analyse 5. On tire ensuite à la trompe à eau par l'intermédiaire de l'embout 6 qu'on a préalablement vissé en place, de manière à éliminer le liquide du filtre et à ne laisser que les micro-organismes sur celui-ci. Puis on ajoute une quantité connue de milieu nutritif choisi pour favoriser la croissance sélective d'un type déterminé de micro-organisme et un substrat fluorogénique. Puis on répète ces opérations de manière à garnir, à volonté, une partie ou la totalité des autres cuvettes d'analyse 5 disponibles sur le plateau. On remonte le plateau 2 ainsi garni dans l'appareil et le cale en place au moyen du verrou 13, après quoi on positionne et ajuste en place le couvercle 3 grâce à l'ergot 18 et on le fixe en replaçant la clavette 19. On règle la température du plateau 1 au moyen d'un courant de liquide à température constante, par exemple de l'eau à 37 °C en provenance d'un thermostat non représenté et alimentant ledit plateau par les tubulures 11 et 12. Puis on sélectionne, au moyen de touches (non représentées au dessin) de l'horloge 40, les différents paramètres opératoires commandés par celle-ci : heure zéro de l'expérience, nombre d'éclairs par mesure, intensité des éclairs, temps de succession des éclairs, nombre de mesures dans le temps et délais entre celles-ci, etc. Puis, toujours sur l'horlóge, on enclenche la commande de mise en marche de l'analyse, les opérations qui en résultent étant les suivantes : Au temps zéro, fixé comme indiqué, le moteur 4 commandé par l'horloge 40 fait tourner le plateau 2 (par l'intermédiaire de son couvercle 3) de manière à amener un premier échantillon en position correcte pour une mesure de fluorescence, c'est-à-dire, à mettre l'une des cuvettes 5 en regard des systèmes optiques d'excitation et de détection. La lampe flash 30, sur commande de l'horloge, fournit le nombre d'éclairs demandés, à l'intensité choisie et aux intervalles sélectionnés (de l'ordre de la seconde ou de la fraction de seconde) et, avec le léger décalage propre au phénomène de fluorescence, le signal de fluorescence est détecté (après chaque éclair), amplifié dans l'ampli 47, intégré dans l'intégrateur 42 et envoyé, simultanément avec le signal de référence, dans le calculateur 44 dont le signal de sortie est affiché en 45, de préférence sous forme logarithmique, la conversion du signal en valeurs logarithmiques étant effectuée par ledit calculateur 44. Il est bien entendu que le calculateur peut fournir des résultats (à l'affichage) indépendants pour chaque éclair ou, mieux, il en fait la moyenne, celle-ci seule étant affichée. Il est aussi bien entendu que, sur commande des circuits de l'horloge (micro-processeur), l'intégrateur 42 peut n'intégrer qu'une portion définie du signal photo-détecté et ceci après un temps donné réglable en fonction des paramètres de fluorescence de la substance fluorescente étudiée. Il est en effet évident qu'on peut, par ce biais, distinguer deux substances fluorescentes différentes, même mélangées entre elles, étant donné qu'avec un fluorochrome commun, les complexes résultant de l'union de celui-ci avec deux micro-organismes différents peuvent avoir des courbes de fluorescences différentes l'une de l'autre. Le présent fluorimètre peut donc se comporter, suivant les réglages de ses circuits de commande, comme un analyseur du contenu chromatique du signal de fluorescence aussi bien qu'un appareil pour mesurer l'énergie totale du signal de fluorescence émis.

Les valeurs de fluorescence du premier échantillon au temps zéro étant enregistrées, l'échantillon n° 2 est mis en place toujours par rotation du plateau sur commande de l'horloge 40 et la séquence des opérations ci-dessus se répète, et ainsi de suite pour les échantillons restants. La mesure des échantillons au temps zéro peut être effectuée très rapidement, par exemple en quelques secondes pour un plateau comportant de six à dix cuvettes 5. On notera encore à ce propos, que la rotation du plateau 2 est assurée, au moment voulu, alternativement dans un sens ou dans l'autre, ceci afin d'éviter, le cas échéant, que les tuyaux souples amenant le liquide thermostatisé aux canalisations 11 et 12 ne subissent des torsions exagérées.

Puis, toujours sous contrôle de l'horloge, l'incubation des cultures dans chaque cuvette se poursuit et, après un temps donné (quelques minutes, 1/2 h, 1 h, par exemple), les mesures sont refaites et les résultats enregistrés, par exemple sous forme d'un second point sur un enregistreur à papier. La succession des points obtenus après un certain nombre de mesures forme alors, pour chaque échantillon, une courbe illustrant la croissance des micro-organismes avec le temps. S'il s'agit de cultures pures, on peut, par référence aux résultats d'analyses traditionnelles, mettre en rapport les résultats des courbes de fluorescence avec les concentrations effectives des micro-organismes considérés. On peut ainsi obtenir des courbes standard pour chaque micro-organisme considéré. On va voir, dans les exemples qui suivent, comment on réalise, pratiquement, de telles courbes standard pour certains micro-organismes connus et comment on utilise ces courbes pour l'analyse d'un échantillon contaminé. Par ailleurs, l'utilisation du fluorimètre pour la mise en œuvre des seconde et troisième applications de l'invention est absolument similaire et évidente pour l'homme de métier sur la base des explications qui précèdent. Des exemples concrets de telles applications sont également fournis plus loin.

Exemple 1

Courbes de fluorescence avec Escherichia coli

On a utilisé un fluorimètre conforme à la description qui précède comportant une lampe excitative à Xenon type FX-198-U (EG & G Electro-optics Co., Salem, Mass. USA) et un photodétecteur de type 1P28. Les filtres étaient destinés à laisser passer, à l'excitation, les longueurs d'onde $\lambda = 384$ nm, et à l'émission, les longueurs d'ondes $\lambda = 492$ nm. Comme échantillon d'étalonnage, on a utilisé les suspensions de concentrations suivantes, par ml, d'Escherichia coli : A $10^7$ ; B $10^6$ ; C $10^5$ ; D $10^4$ ; E zéro (contrôle). On a placé 0,1 ml de la suspension A sur une membrane filtrante disposée dans la cuvette du plateau du fluorimètre puis, après avoir tiré au vide de 10-12 Torr pendant quelques secondes, on a ajouté 0,1 ml de solution nutritive (gélose de Mc Conkey, dans l'eau à 52 g/l) et 0,1 ml d'une solution à 0,1 % de 4-méthylumbelliféryl-β-D-glucoside en solution de Ringer. La membrane poreuse utilisée au fond de la cuvette était une membrane « Millipore Swinnex » (pores 0,45 μ) de 13 mm de diamètre. Puis on a répété la même opération et chargé les autres cuvettes du plateau avec les suspensions B, C, D et E et les réactifs correspondants. On a alors mis en marche le fluorimètre aux conditions opératoires suivantes : température du plateau : 37 °C ; nombre de flashes par mesure et par échantillon : 3, séparés par 0,5 sec ; énergie d'un éclair : 0,1 joule ; durée de l'éclair : 10 μsec ; période entre les mesures : 1 h ; temps de l'analyse : 8 h. Les résultats des mesures ont été enregistrés par une imprimante numérique de type courant et sont rassemblés sous forme d'un graphique représenté à la fig. 3. Sur ce graphique, on a porté les heures en abcisses et le signal de fluorescence en unités arbitraires en ordonnées. Les courbes relatives aux échantillons sont désignées par leur lettre respective. On voit sur ce graphique que, après un certain temps de latence au cours duquel la fluorescence diminue (ceci se produit également pour le contrôle dont la fluorescence, en principe nulle, constitue le bruit de fond de l'appareil), le signal croît régulièrement jusqu'à atteindre un palier (point de saturation). Ces courbes constituent donc des courbes étalon grâce auxquelles il est possible d'identifier, par comparaison, dans un échantillon quelconque, la présence d'Escherichia coli et d'évaluer sa concentration d'origine dans ledit échantillon.

## Exemple 2

Courbes de fluorescence avec Salmonella shigella

On a procédé de manière similaire à ce qui est décrit à l'exemple 1 au moyen d'échantillons A', B', C', D' et E' contenant Salmonella shigella aux concentrations par ml respectives suivantes : A' $10^7$ ; B' $10^6$ ; C' $10^5$ ; D' $10^4$ ; E' zéro (contrôle). On a utilisé comme milieu nutritif 0,1 ml d'une solution de « Gelose SS » à 60 g/l et le même indicateur fluorescent que dans l'exemple précédent. Puis on a procédé aux mesures avec le même fluorimètre et dans les mêmes conditions opératoires que celles de l'exemple précédent. Les résultats obtenus sont présentés sous forme du graphique de la fig. 4 où les lettres désignent, respectivement, les courbes de réponse des différents échantillons analysés. On voit que la forme générale de ces courbes est bien distincte de celles de l'exemple précédent.

## Exemple 3

Analyse d'un échantillon contaminé simultanément par Escherichia coli et Salmonella shigella

On a préparé un échantillon de lait contaminé en mélangeant dans celui-ci des portions de cultures des deux micro-organismes sus-mentionnés. On a prélevé 10 ml de ce lait contaminé et on l'a dégraissé dans une centrifugeuse Haereus de laboratoire à 3 000 rpm pendant 10 min. On a prélevé 4 échantillons de lait écrémé de 0,1 ml et on les a déposés dans quatre des cuvettes d'un fluorimètre analyseur tel que décrit ci-dessus, le fond de chaque cuvette ayant été préalablement garni d'une membrane filtrante « Millipore Swinnex » de 0,45 μ.

Puis, après avoir séché les échantillons par aspiration, comme déjà décrit, on a déposé sur deux d'entre-eux (F et G) 0,1 ml de la solution de « Gélose de Mc Conkey » (voir Exemple 1) et, sur les deux autres (H et I), 0,1 ml de solution de « Géloses SS » (voir Exemple 2). On a encore ajouté l'indicateur de fluorescence et on a procédé exactement comme dans les Exemples précédent.

Les mesures ont donné les résultats suivants : les échantillons F et G ont donné naissance à une courbe de fluorescence de forme semblable à celles de la fig. 3, cette courbe se situant approximativement à équi-distance entre les courbes C et D ; on en a donc conclu que la concentration initiale dans le lait écrémé à analyser d'Escherichia coli était comprise entre 10 000 et 100 000 bactéries/ml.

Les échantillons H et I ont donné des courbes se situant légèrement au-dessus de la courbe B' de la Fig. 4. On en a donc conclu que le lait contaminé contenait un peu plus d'un million de Salmonella par ml.

Il reste bien entendu que les tests ci-dessus sont applicables à d'autres micro-organismes mis en cultures sur d'autres milieux nutritifs ; à titre d'exemples on peut citer les Streptocoques élevés en milieu de Litsky (acide + violet d'éthyle) — les Staphylocoques en milieu de Chapman (mannitol + agar-sel) — Champignons et moisissures sur gélose « Fungiphil » (Biomérieux) — levures sur gélose de Sabouraud, etc...

## Exemple 4

Réalisation d'un antibiogramme

On a utilisé un fluorimètre conforme à celui décrit dans la présente spécification pour la détermination simultanée du pouvoir antibiotique de quatre substances différentes : la pénicilline (1), la gentamicine (2), l'ampicilline (3) et l'érythromycine (4).

Pour ce faire, on a placé 0,1 ml d'une suspension à $10^5$ germes/ml de staphylococcus aureus sur une membrane poreuse de 13 mm de diamètre (membrane « Millipore Swinnex », pores de

0,45 μ) placée dans une des alvéoles du plateau de mesure du fluorimètre. On a tiré à la trompe à eau pour sécher la membrane puis on a ajouté successivement 0,1 ml de milieu de « Chapman et Stone » (solution nutritive), 0,1 ml d'une solution de 4-méthylumbelliféryl-β-D-glucoside à 0,1 % dans de la solution de Ringer et 0,1 ml d'une solution de l'antibiotique (1) à 1 μg/ml. On a chargé identiquement 4 autres alvéoles dudit plateau, mais en ajoutant sur chacun de 3 d'entre-eux la même quantité d'un des antibiotiques (2), (3) et (4) restants. La cinquième cuvette n'a pas reçu d'antibiotiques (témoin).

On a ensuite mis le fluorimètre en marche et travaillé comme décrit en détail à l'Exemple 1 avec les différences suivantes : périodes entre les mesures 1/2 h, temps total de l'analyse 5 h. Comme décrit à l'Exemple 1, les résultats ont été enregistrés automatiquement sur un ruban de papier et sont consignés schématiquement sur le graphique de la fig. 5, ce graphique étant établi suivant les mêmes critères que ceux des figs. 3 et 4. Les courbes représentant la réponse de chacun des échantillons sont désignées par les numéros respectifs désignant, comme indiqué plus haut, les antibiotiques étudiés. Le témoin n'est pas numéroté.

On voit, par les résultats du graphique de la fig. 5, que la pénicilline n'a qu'un effet insignifiant sur la croissance de S. aureus, tandis que les autres antibiotiques inhibent nettement cette croissance et, plus particulièrement, l'érythromycine.

Exemple 5

Réalisation d'un test de Ames

On a procédé exactement comme décrit aux Exemples précédents à partir des réactifs suivants : suspension de Salmonella typhimurium (his-) à $10^5$ germes/ml : 0,1 ml ; solution nutritive (gélose SS salmonella sans histidine) : 0,1 ml ; solution Ringer de 4-méthylumbelliferyl-β-D-glucoside à 0,1 % : 0,1 ml ; substance dont le pouvoir mutagène est à déterminer en solution aqueuse à 1 μg/ml : 0,1 ml. Parmi celle-ci, on a sélectionné la tartrazine (un colorant alimentaire) et le caramel ordinaire (obtenu par pyrolyse du sucre).

On a opéré comme décrit aux exemples précédents (durée du test 6 h) et en examinant les courbes de fluorescence, on a constaté que la fluorescence de l'échantillon contenant la tartrazine avait augmenté au cours du temps tandis que celle de l'échantillon contenant le caramel n'avait pas évolué. On en a donc conclu que la tartrazine présente un pouvoir mutagénique et qu'elle est peut-être cancérigène aux concentrations du test alors que cela n'est pas le cas du caramel.

**Revendications**

1. Méthode pour qualitativement et/ou quantitativement suivre in vivo le développement de micro-organismes croissant sur un milieu de culture, par l'entremise de mesures périodiques de fluorescence dont l'intensité est proportionnelle à la quantité des micro-organismes présents au moment de la mesure, caractérisée par le fait que, lors de l'ensemencement dudit milieu de culture avec lesdits micro-organismes, on ajoute à celui-ci un excès d'une substance fluorogénique ne possédant que peu ou pas de fluorescence propre mais capable de réagir avec lesdits micro-organismes et, par cela, de fournir audit milieu sans porter préjudice au développement de ceux-ci, un agent émetteur de fluorescence, la quantité ajoutée de ladite substance fluorogénique constituant une réserve potentielle suffisante dudit agent fluorescent quel que soit le degré dudit développement au terme desdites mesures périodiques.

2. Application de la méthode de la revendication 1 à la détection, l'identification et/ou le dosage un ou plusieurs types de micro-organismes dans des échantillons de matières organiques ou minérales dans laquelle :

a) on ensemence avec des aliquots de l'échantillon à analyser une série de milieux ou compositions de culture différentes, chacun d'entre ceux-ci étant apte à promouvoir la croissance sélective d'au moins une souche distincte de micro-organismes, contenant au moins une substance fluorogénique pouvant causer, sous l'influence d'une irradiation convenable et en présence de micro-organismes, une émission de fluorescence d'intensité proportionnelle à la quantité de ceux-ci,

b) à intervalles de temps, on irradie ledit milieu de culture et on mesure le degré d'intensité de cette fluorescence dans chaque milieu de culture de manière à observer l'évolution de la croissance desdits micro-organismes et, le cas échéant, le taux de celle-ci,

c) on compare les taux ainsi mesurés avec des taux de croissance standard établis, dans les mêmes milieux et dans les mêmes conditions pour des cultures de chacune des souches à l'état pur, de manière à identifier les micro-organismes soumis à l'analyse, et

d) on utilise les résultats des mesures pour estimer ou calculer statistiquement, par exemple par extrapolation au temps zéro, la quantité des micro-organismes présents dans l'échantillon de départ.

3. Application suivant la revendication 2, caractérisée par le fait que ladite substance provoquant la fluorescence est un substrat fluorogénique.

4. Application suivant la revendication 3, caractérisée par le fait que le composé fluorogénique est choisi parmi les 4-méthylumbelliféryl-β-D-glucopyrannosides.

5. Application suivant la revendication 2, caractérisée par le fait que l'irradiation se fait par impulsions d'une lampe flash, le signal fluorescent résultant étant photodétecté, amplifié, comparé à un signal de référence et le résultat de la comparaison affiché sur un système d'affichage ou d'enregistrement.

6. Application de la méthode de la revendication 1 à la réalisation d'un antibiogramme suivant laquelle :

a) on sélectionne une souche de micro-organismes de développement positif sur un milieu de culture donné et, après y avoir ajouté un substat fluorogénique et un antibiotique à tester, on ensemence celui-ci avec lesdits micro-organismes et le maintient dans des conditions propices à la croissance de ceux-ci,

b) à intervalles de temps, on irradie ledit milieu et on mesure le degré d'intensité de la fluorescence qui en résulte ce qui permet de déterminer la vitesse de croissance desdits micro-organismes,

c) on compare ce taux ainsi mesuré avec celui obtenu de manière similaire au moyen d'une culture exempte de l'antibiotique étudié ce qui permet de déterminer le caractère inhibiteur ou non inhibiteur de celui-ci et son degré d'activité.

7. Application suivant la revendication 6, caractérisée par le fait qu'on opère sur plusieurs échantillons de culture identiques, chacun d'entre eux contenant une concentration différente dudit antibiotique.

8. Application suivant la revendication 6, caractérisée par le fait qu'on opère sur des cultures de micro-organismes différents, chacun d'entre eux contenant une même concentration dudit antibiotique.

9. Application suivant la revendication 6, caractérisée par le fait qu'on opère sur plusieurs échantillons de culture identiques, chacun d'entre-eux contenant un antibiotique différent.

10. Application de la méthode de la revendication 1 à la réalisation du test d'« Ames » pour la détermination du pouvoir cancérigène d'une matière, caractérisée par le fait que,

a) on ensemence de la gélose SS salmonella sans histidine contenant un substrat fluorogénique et la matière à étudier avec une souche de Salmonella thiphimurium (his-) et on maintient l'échantillon dans des conditions favorables au développement des bactéries,

b) à intervalles de temps, on irradie ledit milieu et on mesure l'intensité de la fluorescence éventuellement émise par l'échantillon.

11. Application suivant la revendication 10, caractérisée par le fait qu'on opère sur plusieurs échantillons différents l'un de l'autre, soit par la nature de ladite matière, soit par la concentration de celle-ci dans le milieu de culture.

12. Utilisation, pour réaliser les applications des revendications 2, 6 et 10, d'un fluorimètre comprenant essentiellement un plateau porte-réactifs tournant creusé d'alvéoles destinées à recevoir les échantillons d'analyse et les réactifs nécessaires à celle-ci ; un dispositif optique pour émettre un rayonnement d'excitation par impulsions, ce rayonnement, de longueur d'onde choisie, étant appliqué, lors de la mesure sur les échantillons ; un dispositif détecteur pour capter et diriger la lumière de fluorescence émise par l'échantillon sur un photodétecteur ; et un centre de contrôle et de calcul pour commander les fonctions du fluorimètre et notamment, traiter les signaux détectés et afficher les résultats des mesures, le dispositif d'excitation et ledit plateau étant, pour leur part, asservis audit centre de contrôle de manière que, sur commande de celui-ci, chacune des alvéoles de ce plateau soit amenée en regard des dispositifs excitateur et détecteur et que chaque échantillon soit, tour à tour, irradié par le rayonnement excitateur et le signal de fluorescence détecté, amplifié, traité et affiché par l'entremise dudit centre de contrôle.

13. Utilisation suivant la revendication 12, caractérisée par le fait que le dispositif excitateur du fluorimètre comprend une lampe flash fournissant des éclairs d'énergie réglable pouvant aller jusqu'à environ 1 Ws.

14. Utilisation suivant la revendication 12, caractérisée par le fait que la lumière d'excitation du fluorimètre est dirigée perpendiculairement au milieu fluorescent, de manière à éviter les réflexions parasites en direction du photodétecteur.

15. Utilisation suivant la revendication 12, caractérisée par le fait que les résultats des mesures effectuées sont affichés sur un dispositif enregistreur les imprimant sous forme de valeurs logarithmiques, ces valeurs étant calculées, à partir des signaux analogiques détecés et traités, par ledit centre de contrôle.

**Claims**

1. A method of qualitatively and/or quantitatively, in vivo, monitor the growth of microorganisms growing on a culture medium by means of staggered fluorescent measurements whose intensity is in proportion to the amount of microorganisms present at the measurement time, characterized in adding to said culture medium at the time of sowing thereof with said microorganisms an excess of a fluorogenic substance with little or no intrinsic fluorescence but capable of reacting with said microorganisms, whereby a fluorescent emitter agent is produced without inhibiting the growth of the microorganisms, the quantity of said added fluorogenic substance constituting a sufficient potential reserve of said fluorescent agent, whatever the extent of said growth at the end of said staggered measurements.

2. Application of the method of claim 1 to the dectection, the identification and/or the determination of one or more kinds of microorganisms in samples of organic or mineral materials, whereby :

a) one sows a plurality of different culture media or compositions with aliquots of the analytical samples, each medium enabling the selective growth of at least one distinct strain of microorganism and containing at least one fluorogenic substance capable of providing, when properly irradiated in the presence of the microorganisms, a fluorescence emission of which the intensity is proportional to the amount thereof,

b) one irradiates at staggered periods said culture medium and one measures the intensity of this fluorescence in each culture medium so as to monitor the growth evolution of said microorganisms and, occasionally, the rate thereof,

c) one compares the measured rates with standard growth rates achieved with the same media and under the same conditions for cultures of each independent strain in the pure state in order to identify the microorganisms subjected to analysis, and

d) one utilizes the measured results to evaluate or statistically compute, for instance by extrapolating to time zero, the quantity of microorganisms present in the starting sample.

3. Application according to claim 2, characterized in that said fluorescence inducing substance is a fluorogenic substrate.

4. Application according to claim 3, characterized in that the fluogenic compound is selected from 4-methylumbelliferyl-β-D-glucopyranosides.

5. Application according to claim 2, characterized in that the irradiation includes pulses from a flash tube, the resulting fluorescent signal being photodetected, amplified, compared with a reference signal and the result of the comparison is displayed on a display or recorder system.

6. Application of the method according to claim 1 for performing an antibiogram according to which :

a) one selects a microorganism strain having positive growth characteristics in a given culture medium and, after adding thereto a fluorogenic substrate and a antibiotic to be tested, one sows said medium with said microorganisms and one keeps said medium under suitable conditions for the growing thereof,

b) at staggered periods of time one irradiates said medium and one measures the intensity of the resulting fluorescence which enables to determine the growth rate of said microorganisms,

c) one compares this measured rate with the rate measured similarly in the case of a culture deprived from the antibiotic under study which enables to determine the inhibitory or non inhibitory character of the latter and its activity.

7. Application according to claim 6, characterized in that one works with several identical culture samples, each of them containing a different concentration of said antibiotic.

8. Application according to claim 6, characterized in that one works with cultures of different microorganisms, each of them containing the same concentration of said antibiotic.

9. Application according to claim 6, characterized in that one works with several identical culture samples, each containing a different antibiotic.

10. Application of the method according to claim 1 for performing an « Ames » test for the determination of the carcinogenic power of a material, characterized in that,

a) one sows a sample of agar SS salmonella without histidine containing a fluorogenic substrate and the material to be studied with a strain of Salmonella thiphimurium (his-) and one keeps the sample under conditions suitable for the growth of the bacteria,

b) at staggered time periods, one irradiates said medium and one measures the intensity of the fluorescence possibly emitted by the sample.

11. Application according to claim 10, characterized in that one works on several samples which may differ from each other, either by the nature of said material, or by the concentration thereof in said culture medium.

12. Utilization for achieving the application of claim 2, 6 and 10 of a fluirometer comprising essentially a reactant carrier turntable provided with recesses for receiving the analytical samples and the reagents required thereto ; an optical device for generating a pulsed excitation radiation, this radiation being of selected wavelength to be applied to the sample during the measurement ; a detector device for collecting and directing fluorescent light emitted from said sample on a photodetector ; and a controlling and computing device for controlling the functions of the fluorimeter and, namely, for treating the detected signals and provide measurements readouts, the exciting device and said turntable being themselves connected to said controlling device so that, upon control from the latter, each recess of the turntable is brought in facing relationship with the excitation and detector devices and that each sample is irradiated in turn by the exciting radiation and the detected fluorescent signal is amplified, processed and displayed by means of said controlling device.

13. Utilization according to claim 12, characterized in that the excitation device of the fluorimeter comprises a flash tube providing controlled energy flashes up to about 1 Ws.

14. Utilization according to claim 12, characterized in that the light of excitation of the fluorimeter is directed perpendicularly to the fluorescent medium, so as to avoid parasitic reflexions in the direction of the photodetector.

15. Utilization according to claim 12, characterized in that the results of the effected measurements are displayed on a recorder device which prints them in the form of logarithmic values, such values being calculated from analog signals detected and processed by said controlling device.

## Ansprüche

1. Verfahren zum qualitativen und/oder quantitativen in vivo-Beobachten der Entwicklung von auf einem Nährboden wachsenden Mikroorganismen durch Vermittlung periodischer Fluoreszenz-Messungen, deren Intensität proportional der Quantität der im Moment der Messung vorhandenen Mikroorganismen ist, dadurch gekennzeichnet, daß man beim Einsäen des Nährbodens mit den Mikroorganismen diesem einen Überschuß einer fluorerzeugenden Substanz beimengt, die wenig oder keine Eigenfluorenszenz, aber die Fähigkeit aufweist, mit den Mikroorganismen zu reagieren und dadurch dem Nährboden ohne Nachteil für die Entwicklung dieser ein Fluorenszenz ausstrahlendes Mittel zu liefern, wobei die beigemengte Quantität der fluorerzeugenden Substanz einen potentiellen, ausreichenden Vorrat für das fluoreszierende Mittel darstellt, wie hoch auch der Entwicklungsgrad am Ende der periodischen Messungen sein mag.

2. Anwendung des Verfahrens nach Anspruch 1 zur Feststellung, Identifizierung und/oder Dosierung einer oder mehrerer Arten von Mikroorganismen in Proben organischer oder mineralischer Stoffe, bei denen

a) man mit Aliquoten der zu analysierenden Probe eine Reihe verschiedener Nährböden oder -zusammensetzungen einsät, wobei jede unter diesen das wahlweise Anwachsen mindestens eines bestimmten Stammes von Mikroorganismen fördern kann, und die mindestens eine fluorerzeugende Substanz enthalten, welche unter dem Einfluß einer angemessenen Bestrahlung und in Anwesenheit von Mikroorganismen eine Fluoreszenz-Ausstrahlung mit einer der Quantität dieser proportionalen Menge hervorrufen kann,

b) man den Nährboden in Zeitintervallen bestrahlt und den Grad der Intensität dieser Fluoreszenz in jedem Nährboden dergestalt mißt, daß die Fortentwicklung des Anwachsens der Mikroorganismen und gegebenenfalls der Grad desselben beobachtet wird,

c) man die derart gemessenen Grade mit den üblichen Standard-Wachstumsgraden bei gleichen Nährböden und unter gleichen Bedingungen für Kulturen jedes der Stämme in reinem Zustand vergleicht, um so die der Analyse unterworfenen Mikroorganismen zu identifizieren, und

d) man die Meßergebnisse verwendet, um statistisch, beispielsweise durch Extrapolation zur Zeit Null die Quantität der in dem Ausgangsmuster enthaltenen Mikroorganismen zu schätzen oder zu berechnen.

3. Anwendung nach Anspruch 2, dadurch gekennzeichnet, daß die die Fluorenszenz hervorrufende Substanz ein fluorerzeugendes Substrat ist.

4. Anwendung nach Anspruch 3, dadurch gekennzeichnet, daß die fluorerzeugende Verbindung aus den 4-Methylumbelliferyl-ß-D-glucopyrannosiden gewählt wird.

5. Anwendung nach Anspruch 2, dadurch gekennzeichnet, daß die Bestrahlung durch Impuls einer Blitzlichtlampe stattfindet, wobei das sich ergebende fluoreszierende Signal photodetektiert, verstärkt, mit einem Bezugssignal verglichen und das Ergebnis des Vergleichs auf einem Anzeige- oder Registriersystem angezeigt wird.

6. Anwendung des Verfahrens nach Anspruch 1 auf die Herstellung eines Antibiogramms, nach welchem

a) man einen Stamm von Mikroorganismen mit positiver Entwicklung auf einem gegebenen Nährboden auswählt und nach Zugabe eines fluorerzeugenden Substrats und eines zu prüfenden Antibiotikums dieses mit den Mikroorganismen besät und es unter für das Anwachsen dieser günstigen Bedingungen hält,

b) man den Nährboden in Zeitintervallen bestrahlt und den sich daraus ergebenden Intensitätsgrad der Fluoreszenz mißt, was ermöglicht, die Wachstumsgeschwindigkeit der Mikroorganismen zu bestimmen,

c) man diesen derart gemessenen Grad mit dem in ähnlicher Weise mittels einer von dem Antibiotikum freien Kultur erhaltenen vergleicht, was ermöglicht, die inhibierende oder nicht inhibierende Eigenschaft dieses und seinen Aktivitätsgrad festzustellen.

7. Anwendung nach Anspruch 6, dadurch gekennzeichnet, daß man auf mehreren Proben gleicher Kulturen arbeitet, wobei jede dieser eine unterschiedliche Konzentration des Antibiotikums enthält.

8. Anwendung nach Anspruch 6, dadurch gekennzeichnet, daß man auf Kulturen unterschiedlicher Mikroorganismen arbeitet, wobei jede dieser eine gleiche Konzentration des Antibiotikums enthält.

9. Anwendung nach Anspruch 6, dadurch gekennzeichnet, daß man auf mehreren Proben gleicher Kulturen arbeitet, wobei jede dieser ein unterschiedliches Antibiotikum enthält.

10. Anwendung des Verfahrens nach Anspruch 1 auf die Ausführung des « Ames » -Tests zur Bestimmung der krebserzeugenden Fähigkeit eines Stoffes, dadurch gekennzeichnet, daß

a) man SS Salmonellen-Gelose ohne Histidin, die ein fluorerzeugendes Substrat und den zu untersuchenden Stoff enthält, mit einem Stamm von Salmonella thiphimurium (his-) besät und die Probe unter für die Entwicklung von Bakterien günstigen Bedingungen hält,

b) man den Nährboden in Zeitintervallen bestrahlt und die Intensität der gegebenenfalls von der Probe ausgestrahlten Fluoreszenz mißt.

11. Anwendung nach Anspruch 10, dadurch gekennzeichnet, daß man auf mehreren der Natur des Stoffes oder seiner Konzentration in diesem Nährboden nach voneinander verschiedenen Proben arbeitet.

12. Verwendung eines Fluormessers zur Ausführung der Anwendungen gemäß Anspruch 2, 6 und 10, der im wesentlichen eine Reagenzien tragende Drehplatte mit ausgehöhlten Zellen, welche die Analyseproben und die für diese erforderlichen Reagenzien aufnehmen sollen; eine optische Einrichtung zum Ausstrahlen einer Erregerstrahlung durch Impulse, wobei diese Ausstrahlung mit ausgewählter Wellenläge beim Messen auf die Proben angelegt wird; eine Detektoreinrichtung zum Einfangen und Ausrichten des von der Probe auf einen Photodetektor ausgestrahlten Fluorenszenzlichts; und ein Kontroll- und Berechnungszentrum zum Steuern der Funktionen des Fluormessers und insbesondere zum Behandlen der festgestellten Signale und zum Anzeigen der Ergebnisse der Messungen umfaßt, wobei die Erregereinrichtung und die Platte ihrerseits das Kontrollzentrum dergestalt steuern, daß auf Befehl desselben jede der Zellen dieser Platte gegenüber Erreger- und Feststelleinrichtungen geführt wird, und daß jede Probe nach und nach durch die Erregerstrahlung bestrahlt wird und das Fluoreszenzsignal durch Einsetzen des Kontrollzentrums festgestellt, verstärkt, behandelt und angezeigt wird.

13. Verwendung nach Anspruch 12, dadurch gekennzeichnet, daß die Erregereinrichtung des Fluormessers eine Blitzlichtlampe umfaßt, die regelbare Energieblitze bis zu ungefähr 1 Ws liefert.

14. Verwendung nach Anspruch 12, dadurch gekennzeichnet, daß das Erregerlicht des Fluormessers senkrecht zum fluoreszierenden Nährboden gerichtet ist, um die parasitären Reflexionen in Richtung des Photodetektors zu vermeiden.

15. Verwendung nach Anspruch 12, dadurch gekennzeichnet, daß die Ergebnisse der durchgeführten Messungen auf einer Registriereinrichtung angezeigt werden, die sie in Form von logarithmischen Werten druckt, wobei diese Werte aus analogen Signalen, die durch das Kontrollzentrum entdeckt und behandelt werden, berechnet werden.

FIG. I

0 054 001

FIG. 2

FIG. 3

FIG. 4

FIG. 5